(19) Europäisches Patentamt
European Patent Office
Office européen des brevets

(11) Numéro de publication: **0 262 062 B1**

# (12) FASCICULE DE BREVET EUROPEEN

(45) Date de publication du fascicule du brevet: **23.08.89**

(51) Int. Cl.⁴: **C07C 37/07**, C07C 39/32, C07C 39/34, C07C 39/36

(21) Numéro de dépôt: **87420252.6**

(22) Date de dépôt: **24.09.87**

(54) **Procédé de préparation de chlorophénols.**

(30) Priorité: **25.09.86 FR 8613556**

(43) Date de publication de la demande: **30.03.88 Bulletin 88/13**

(45) Mention de la délivrance du brevet: **23.08.89 Bulletin 89/34**

(84) Etats contractants désignés: **AT BE CH DE ES FR GB GR IT LI LU NL SE**

(56) Documents cités:
**EP-A- 0 123 233**
**FR-A- 958 804**
**US-A- 3 760 010**

(73) Titulaire: **RHONE-POULENC CHIMIE, 25, quai Paul Doumer, F-92408 Courbevoie Cédex(FR)**

(72) Inventeur: **Desmurs, Jean, La Jonquière Route de Ternay, Communay F-69360 St-Symphorien d'Ozon(FR)**
Inventeur: **Ratton, Serge, Hameau de Belmont, Vaulx Milieu F-38090 Villefontaine(FR)**

(74) Mandataire: **Vignally, Noel et al, Rhône-Poulenc Interservices Service Brevets Chimie Centre de Recherches des Carrières B.P. 62, F-69192 Saint-Fons Cédex(FR)**

**EP 0 262 062 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de préparation de chlorophénols.

Les chlorophénols sont généralement obtenus par hydrolyse de polychlorobenzènes ou par chloration du phénol.

Lors de la chloration du phénol, on obtient généralement à chaque stade de la chloration, plusieurs isomères de position et il est difficile de préparer de manière sélective un seul composé déterminé.

Lorsque l'on atteint le stade des trichlorophénols, tétrachlorophénols et pentachlorophénol, les mélanges de chloration son très complexes.

La demanderesse a mise en évidence, dans ces mélanges de chloration, des cétones cycliques insaturées comportant une substitution gem-dichlorée.

L'objet de la présente invention est un procédé de préparation de polychlorophénols, caractérisé en ce que :

- l'on traite une ou plusieurs chlorocyclohexadiènones de formules (I) ou (II) :

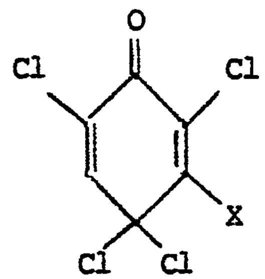

(I)

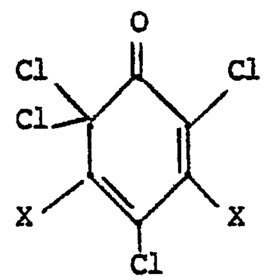

(II)

dans desquelles :

. X représente un atome de chlore ou un atome d'hydrogène,

. les deux symboles X de la formule (II) ne représentent pas simultanément un atome de chlore,

- en présence d'un acide protonique fort et/ou d'un acide de Lewis.

Par acide protonique fort, on entend dans le présent texte un acide protonique ayant une fonction d'acidité Ho inférieure ou égale à -5.

Comme exemples non limitatifs de tels acides forts, on peut citer l'acide sulfurique, l'acide perchlorique, l'acide trifluorométhanesulfonique, l'acide chlorosulfonique, l'acide fluorosulfonique, l'acide pyrosulfurique ainsi que les résines acides comportant des groupements fluorosulfoniques.

On assimilera également dans ce qui suit aux acides protoniques forts les formes acides des aluminosilicates, les formes acides des argiles et les silices.

Parmi les aluminosilicates acides, on peut citer notamment les zéolithes et les tamis moléculaires et parmi les argiles acides on peut citer notamment les bentonites.

Par acide de Lewis, on entend dans le présent texte, selon la définition usuelle, des composés accepteurs de doublets électroniques.

On peut utiliser notamment les acides de Lewis cités dans l'ouvrage édité par G.A. OLAH "Friedel-Crafts and related Reactions", tome I, pages 191 à 197 (1963).

Les acides de Lewis pouvant servir dans le procédé, sont plus particulièrement les halogénures d'éléments des groupes 3a, 4a, 5a, 1b, 2b, 3b, 4b, 5b, 6b, 7b et 8 de la classification périodique des éléments, qui sont liquides ou solides dans les conditions opératoires, tels que les chlorures, bromures, fluorures et iodures d'aluminium, d'étain, de phosphore, d'antimoine, d'arsenic, de bismuth, de titane, de tantale, de tellure, de sélénium, de zirconium, de vanadium, de samarium, de nobium, de tungstène, de platine, de molybdène, de fer, de cobalt, de nickel, de zinc et de cadmium.

Comme exemples spécifiques de tels halogénures, on peut citer le chlorure d'aluminium, le chlorure ferrique, le tétrachlorure de titane, le tétrachlorure de zirconium, le chlorure de platine, le trichlorure de vanadium, le chlorure de samarium, le chlorure de tellure, le chlorure de sélénium, le pentafluorure d'antimoine, l'hexachlorure de tungstène, le trichlorure de bismuth, le chlorure stannique.

Les cyclohexadiènones gem-dichlorées de formules (I) et (II) qui servent à préparer les tétrachlorophénol et pentachlorophénol, sont : la tétrachloro-2,4,4,6 cyclohexadiène-2,5 one ; la tétrachloro-2,2,4,6 cyclohexadiène-3,5 one ; la pentachloro-2,3,4,4,6 cyclohexadiène-2,5 one ; la pentachloro-2,2,4,5,6 cyclohexadiène-3,5 one et la pentachloro-2,2,3,4,6 cyclohexadiène-3,5 one.

Les polychlorophénols obtenus sont essentiellement le tétrachloro-2,3,4,6 phénol et le pentachlorophénol. On peut également obtenir parfois du trichloro-2,4,6 phénol.

Le procédé consiste essentiellement à chauffer la chlorocyclohexadiènone gem-dichlorée avec l'acide protonique fort et/ou l'acide de Lewis à une température généralement égale ou supérieure à 50°C.

De préférence la température à laquelle est mis en oeuvre le procédé de l'invention est comprise entre 60°C et 150°C.

On peut opérer en présence ou en l'absence de solvant.

Lorsque l'on utilise un solvant, celui-ci peut appartenir à des familles très diverses, dans la mesure où il ne réagit pas avec le substrat ou le catalyseur

Ainsi on peut citer des éthers tels que l'oxyde de diisopropyle, des acides carboxyliques tels que l'acide acétique, des hydrocarbures nitrés tels que le nitrobenzène, des hydrocarbures chlorés tels que le trichloroéthylène, des amides tels que le diméthylformamide, le diméthylacétamide.

On peut également utiliser comme solvant un ou plusieurs chlorophénols, notamment le trichloro-2,4,6 phénol, le tétrachloro-2,3,4,6 phénol et le pentachlorophénol.

La quantité d'acide protonique ou d'acide de Lewis représente généralement, en moles par rapport à 1 mole de cyclohexadiènone gem-dichlorée, au moins 0,05. La limite supérieure de ce rapport molaire n'est pas critique, mais il n'est habituellement pas nécessaire d'avoir un rapport molaire acide protonique et/ou acide de Lewis/cyclohexadiènone gem-dichlorée supérieur à 10.

De préférence, ce rapport molaire sera compris entre 0,2 et 5. Généralement on utilisera un rapport molaire acide protonique et/ou acide de Lewis/cyclohexadiènone gem-dichlorée plus élevé lorsque l'on mettra en oeuvre le procédé en milieu solvant.

EXEMPLES 1 à 8 - (Essais sans solvant)

Dans un réacteur en verre de 1 cm3 comportant une agitation, on charge :
- tétrachloro-2,4,4,6 cyclohexadiène-2,5 one : 0,23 g (0,001 mole)
- acide protonique fort ou acide de Lewis : 0,0005 mole (sauf indication contraire).

On chauffe à la température choisie, pendant un temps donné.

On analyse la masse réactionnelle par chromatographie en phase liquide à l'aide d'une double détection U.V. et ampérométrie.

On dose les chlorophénols formés et la tétrachlorocyclohexadiénone restant éventuellement.

Le tableau (I) ci-après indique la nature de l'acide utilisé, la température et la durée de la réaction ainsi qu le taux de transformation (TT) de la tétrachlorocyclohexadiènone et les rendements (RT) en chlorophénols par rapport à la tétrachlorocyclohexadiènone transformée.

Tableau (I)

| Essais | Acide utilisé | Température °C | Durée | TT % TCCD | RT % en TCP | RT % en TTCP | RT % en PCP |
|---|---|---|---|---|---|---|---|
| Témoin A | Néant | 70 | 8 h | 0 | 0 | 0 | 0 |
| Témoin B | Néant | 125 | 8 h | 0 | 0 | 0 | 0 |
| Exemple 1 | $AlCl_3$ | 70 | 16 h | 100 | 6 | 80 | 9 |
| Exemple 1 bis | $AlCl_3$ | 70 | 10 min | 100 | 6 | 80 | 9 |
| Exemple 2 | $CF_3SO_3H$ | 70 | 16 h | 100 | 4 | 94 | 2 |
| Exemple 2 bis | $CF_3SO_3H$ | 70 | 10 min | 100 | 4 | 94 | 2 |
| Exemple 3 | $ZrCl_4$ | 70 | 8 h | 100 | 19 | 78 | 3 |
| Exemple 4 | $MoCl_3$ | 70 | 8 h | 100 | 45 | 13 | 0 |
| Exemple 5 | $SmCl_3$, 6 $H_2O$ | 125 | 8 h | 91 | 17,4 | 26,1 | 4,3 |
| Exemple 6 | Zéolithe L 0,25 g | 70 | 8 h | 90 | 14,3 | 75,8 | 0 |
| Exemple 7 | Bentonite sodique 0,25 g | 70 | 8 h | 91 | 19,6 | 12 | 0 |
| Exemple 8 | Tamis moléculaire 0,50 g | 125 | 8 h | 91 | 27 | 26 | 0 |

TCCD = tétrachlorocyclohexadiènone
TCP = trichloro-2,4,6 phénol
TTCP = tétrachloro-2,3,4,6 phénol
PCP = pentachlorophénol

EXEMPLES 9 à 17 - (Essais en milieu solvant)

On répète les exemples 1 à 8, mais en opérant dans un réacteur de 10 cm3.

On charge :
- tétrachloro-2,4,4,6 cyclohexadiène-2,5 one :0,23 g (0,001 mole)
- acide protonique ou acide de Lewis :0,0025 mole.
- solvant :0,01 mole.

Le tableau (II) ci-après indique la nature de l'acide et du solvant utilisés, la température et la durée de la réaction ainsi que le TT de la tétracyclohexadiènone et les RT en chlorophénols.

Tableau (II)

| Essais | Acide utilisé Solvant utilisé | Tempéra-ture °C | Durée | TT % TCCD | RT % en TCP | RT % en TTCP | RT % en PCP |
|---|---|---|---|---|---|---|---|
| Témoin C | – oxyde de diisopropyle | 70 | 8 h | 0 | 0 | 0 | 0 |
| Exemple 9 | $AlCl_3$ oxyde de diisopropyle | 70 | 8 h | 88,1 | 83,1 | 16,7 | 0 |
| Exemple 10 | $AlCl_3$ acide acétique | 70 | 8 h | 72,3 | 0 | 98,6 | 0 |
| Exemple 11 | $AlCl_3$ nitrobenzène | 70 | 8 h | 100 | 8 | 88 | 2 |
| Exemple 12 | $AlCl_3$ diméthylacétamide | 70 | 8 h | 100 | 60,6 | 26,3 | 5,1 |
| Exemple 13 | $AlCl_3$ propanol-2 | 70 | 8 h | 58,2 | 31,6 | 68,4 | 0 |
| Exemple 14 | $AlCl_3$ trichloroéthylène | 70 | 8 h | 100 | 6 | 11 | 0 |
| Exemple 15 | $CF_3SO_3H$ acide acétique | 70 | 8 h | 100 | 25,3 | 61,6 | 0 |
| Exemple 16 | $CF_3SO_3H$ nitrobenzène | 70 | 8 h | 100 | 14 | 76 | 1 |
| Exemple 17 | $CF_3SO_3H$ trichloroéthylène | 70 | 8 h | 100 | 1 | 77,8 | 0 |

EXEMPLES 18 à 21 - (Essais sans solvant)

On répète les exemples 1 à 8 avec les charges suivantes :
- pentachloro-2,3,4,4,6 cyclohexadiène-2,5 one (PCCD) : 0,266 g (0,001 mole)
- acide protonique fort ou acide de Lewis : 0,0005 mole.

Le tableau (III) ci-après indique la nature de l'acide utilisé, la température et la durée de la réaction ainsi que le TT de la PCCD et les RT en chlorophénols.

Tableau (III)

| Essais | Acide utilisé | Tempéra-ture °C | Durée | TT % PCCD | RT % en TTCP | RT % en PCP |
|---|---|---|---|---|---|---|
| Témoin D | Néant | 70 | 8 h | 12 | 0 | 0 |
| Témoin E | Néant | 125 | 8 h | 13 | 0 | 0 |
| Exemple 18 | $AlCl_3$ | 70 | 8 h | 100 | 12,9 | 79,2 |
| Exemple 19 | $AlCl_3$ | 125 | 8 h | 100 | 9 | 91 |
| Exemple 20 | $CF_3SO_3H$ | 70 | 8 h | 100 | 14 | 82 |
| Exemple 21 | $CF_3SO_3H$ | 125 | 8 h | 100 | 8 | 90 |

PCCD = pentachloro-2,3,4,4,6 cyclohexadiène-2,5 one
TTCP = tétrachloro-2,3,4,6 phénol
PCP = pentachlorophénol

## Revendications

1°) Procédé de préparation de polychlorophénols, caractérisé en ce que :
- l'on traite une ou plusieurs chlorocyclohexadiène-ones de formules (I) ou (II) :

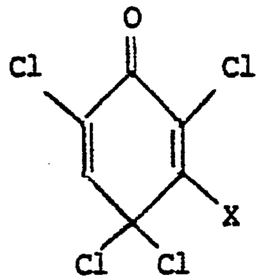
(I)

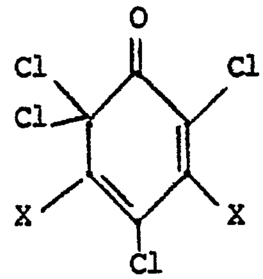
(II)

das desquelles :

. X représente un atome de chlore ou un atome d'hydrogène,

. les deux symboles X de la formule (II) ne représentent pas simultanément un atome de chlore,

- en présence d'un acide protonique fort et/ou d'un acide de Lewis.

2°) Procédé selon la revendication 1, caractérisé en que que l'acide protonique fort est choisi parmi l'acide sulfurique, l'acide perchlorique, l'acide trifluorométhanesulfonique l'acide chlorosulfonique, l'acide fluorosulfonique, l'acide pyrosulfurique ainsi que les résines acides comportant des groupements fluorosulfoniques.

3°) Procédé selon la revendication 1, caractérisé en ce que l'acide fort utilisé est choisi parmi les formes acides des aluminosilicates, les formes acides des argiles et les silices.

4°) Procédé selon la revendication 1, caractérisé en ce que l'acide fort utilisé est choisi parmi les formes acides des aluminosilicates telles que les zéolithes et les tamis moléculaires, les formes acides des argiles telles que les bentonites et les silices.

5°) Procédé selon la revendication 1, caractérisé en ce que l'acide de Lewis est choisi parmi les halogénures d'éléments des groupes 3a, 4a, 5a, 1b, 2b, 3b, 4b, 5b, 6b, 7b et 8 de la classification périodique des éléments, qui sont liquides ou solides dans les conditions opératoires.

6°) Procédé selon l'une des revendications 1 ou 5, caractérisé en ce que l'acide de Lewis est choisi parmi les chlorures, bromures, fluorures et iodures d'aluminium, d'étain, de phosphore, d'antimoine, d'arsenic, de bismuth, de titane, de tantale, de tellure, de sélénium, de zirconium, de vanadium, de samarium, de nobium, de tungstène, de platine, de molybdène, de fer, de cobalt, de nickel, de zinc et de cadmium.

7°) Procédé selon l'une des revendications 1, 5 ou 6, caractérisé en ce que l'acide de Lewis est choisi parmi le chlorure d'aluminium, le chlorure ferrique, le tétrachlorure de titane, le tétrachlorure de zirconium, le chlorure de platine, le trichlorure de vanadium, le chlorure de samarium, le chlorure de tellure, le chlorure de sélénium, le pentafluorure d'antimoine, l'hexachlorure de tungstène, le trichlorure de bismuth, le chlorure stannique.

8°) Procédé selon l'une des revendications 1 à 7, caractérisé en ce que l'on opère à une température égale où supérieure à 50°C et de préférence entre 60°C et 150°C.

9°) Procédé selon l'une des revendications 1 à 8, caractérisé en ce que l'on opère en l'absence de solvant.

10°) Procédé selon l'une des revendications 1 à 8, caractérisé en ce que l'on opère dans un milieu solvant tel qu'un éther, un acide carboxylique, un hydrocarbure nitré, un hydrocarbure chloré, un amide ou un ou plusieurs chlorophénols.

11°) Procédé selon l'une des revendications 1 à 10, caractérisé en ce que le rapport molaire acide protonique et/ou acide de Lewis/cyclohexadiènone gem-dichlorée est de 0,05 à 10 et de préférence de 0,2 à 5.

**Patentansprüche**

1. Verfahren zur Herstellung von Polychlorphenolen, dadurch gekennzeichnet, daß man eine oder mehrere Chlorcyclohexadienone der Formel (I) oder (II)

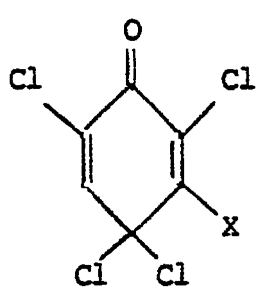
(I)

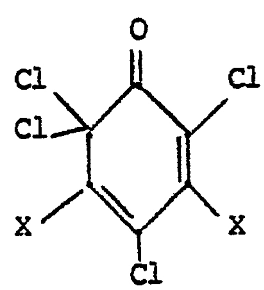
(II)

worin

X ein Chloratom oder ein Wasserstoffatom darstellt, und die beiden Symbole X der Formel (II) nicht gleichzeitig ein Chloratom darstellen,
in Gegenwart einer starken Protonensäure und/oder eine Lewis-Säure behandelt.

2. Verfahren nach Anspruch 1, dadurch gekennzeichnet, daß die starke Protonsäure aus Schwefelsäure, Perchlorsäure, Trifluormethansulfonsäure, Chlorsulfonsäure, Fluorsulfonsäure, Pyroschwefelsäure und Fluorsulfongruppen tragenden sauren Harzen ausgewählt wird.

3. Verfahren nach Anspruch 1, dadurch gekennzeichnet, daß die verwandte starke Säure aus Aluminosilikaten in saurer Form, Tonerden in saurer Form und Silikaten ausgewählt wird.

4. Verfahren nach Anspruch 1, dadurch gekennzeichnet, daß die verwandte starke Säure aus Aluminiumsilikaten in saurer Form, wie Zeolithen und Molekularsieben, Tonerden in saurer Form, wie Bentoniten und Silikaten, ausgewählt wird.

5. Verfahren nach Anspruch 1, dadurch gekennzeichnet, daß die Lewis-Säure aus Halogeniden der Elemente der Gruppen 3a, 4a, 5a, 1b, 2b, 3b, 4b, 5b, 6b, 7b und 8 des Periodensystems der Elemente, die unter den Verfahrensbedingungen flüssig oder fest sind, ausgewählt wird.

6. Verfahren nach einem der Ansprüche 1 oder 5, dadurch gekennzeichnet, daß die Lewis-Säure aus den Chloriden, Bromiden, Fluoriden und Iodiden von Aluminium, Zinn, Phosphor, Antimon, Arsen, Wismuth, Titan, Tantal, Tellur, Selen, Zirkon, Vanadin, Samarium, Niob, Wolfram, Platin, Molybdän, Eisen, Kobalt, Nickel, Zink und Cadium ausgewählt wird.

7. Verfahren nach einem der Ansprüche 1, 5 oder 6, dadurch gekennzeichnet, daß die Lewis-Säure aus Aluminiumchlorid, Eisen-III-chlorid, Titantetrachlorid, Zirkontetrachlorid, Platinchlorid, Vanadiumdichlorid, Samariumchlorid, Tellurchlorid, Selenchlorid, Antimonpentafluorid, Wolframhexachlorid, Wismuthtrichlorid und Zinn-IV-chlorid ausgewählt wird.

8. Verfahren nach einem der Ansprüche 1 bis 7, dadurch gekennzeichnet, daß man bei einer Temperatur von oder oberhalb von 50°C und vorzugsweise zwischen 60°C und 150°C arbeitet.

9. Verfahren nach einem der Ansprüche 1 bis 8, dadurch gekennzeichnet, daß man in Abwesenheit eines Lösungsmittels arbeitet.

10. Verfahren nach einem der Ansprüche 1 bis 8, dadurch gekennzeichnet, daß man in einem Lösungsmittel, wie einem Ether, einer Carbonsäure, einem nitrierten Kohlenwasserstoff, einem chlorierten Kohlenwasserstoff, einem Amid oder einem oder mehreren Chlorphenolen arbeitet.

11. Verfahren nach einem der Ansprüche 1 bis 10, dadurch gekennzeichnet, daß das molare Verhältnis von Protonensäure und/oder Lewis-Säure zu gem-dichloriertem Cyclohexadienon 0,05 bis 10 und vorzugsweise 0,2 bis 5 beträgt.

**Claims**

1. Process for preparing polychlorophenols, characterized in that:
one or more chlorocyclohexadienones of formula (I) or (II):

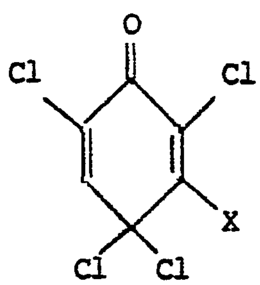

(I)

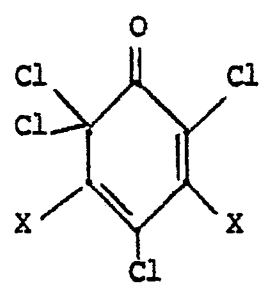

(II)

in which:
X denotes a chlorine atom or a hydrogen atom, the two symbols X in the formula (II) do not simultaneously denote a chlorine atom, are treated in the presence of a strong protonic acid and/or a Lewis acid.

2. Process according to claim 1, characterized in that the strong protonic acid is chosen from sulphuric acid, perchloric acid, trifluoromethanesulphonic acid, chlorosulphonic acid, fluorosulphonic acid, pyrosulphuric acid and also acidic resins containing fluorosulphonic groups.

3. Process according to claim 1, characterized in that the strong acid used is chosen from the acid forms of aluminosilicates, the acid forms of clays, and silicas.

4. Process according to claim 1, characterized in that the strong acid used is chosen from the acid forms of aluminosilicates such as zeolites and molecular sieves, and the acid forms of clays such as bentonites and silicas.

5. Process according to claim 1, characterized in that the Lewis acid is chosen from the halides of elements of Groups 3a, 4a, 5a, 1b, 2b, 3b, 4b, 5b, 6b, 7b and 8 of the Periodic Classification of the elements which are liquid or solid under the working conditions.

6. Process according to either of claims 1 and 5, characterized in that the Lewis acid is chosen from the chlorides, bromides, fluorides and iodides of aluminium, tin, phosphorus, antimony, arsenic, bismuth,

titanium, tantalum, tellurium, selenium, zirconium, vanadium, samarium, niobium, tungsten, platinum, molybdenum, iron, cobalt, nickel, zinc and cadmium.

7. Process according to one of claims 1, 5 or 6, characterized in that the Lewis acid is chosen from aluminium chloride, ferric chloride, titanium tetrachloride, zirconium tetrachloride, platinum chloride, vanadium trichloride, samarium chloride, tellurium chloride, selenium chloride, antimony pentafluoride, tungsten hexachloride, bismuth trichloride and stannic chloride.

8. Process according to one of claims 1 to 7, characterized in that the reaction is performed at a temperature equal to or above 50°C, and preferably between 60°C and 150°C.

9. Process according to one of claims 1 to 8, characterized in that the reaction is performed in the absence of a solvent.

10. Process according to one of claims 1 to 8, characterized in that the reaction is performed in a solvent medium such as an ether, a carboxylic acid, a nitrated hydrocarbon, a chlorinated hydrocarbon, an amide or one or more chlorophenols.

11. Process according to one of claims 1 to 10, characterized in that the mole ratio of protonic acid and/or Lewis acid to gem-dichlorinated cyclohexadienone is from 0.05 to 10, and preferably from 0.2 to 5.